# EUROPÄISCHE PATENTANMELDUNG

(11) **EP 2 428 212 A1**
(43) Veröffentlichungstag der Anmeldung: **14.03.2012**
(21) Anmeldenummer: 11005831.0
(22) Anmeldetag: 15.07.2011
(51) Int. Cl.: A61K 31/7004, A61K 31/195, A61K 31/455, A61K 31/375

(54) **Stoffgemisch, Verwendung und Infusionslösung enthaltend Ribose, Alanin, Nikotinsäure und Ascorbinsäure**

(30) Priorität: 13.09.2010 DE 102010045185
(71) Anmelder: Brøndlund, Marianne, 8462 Harlev (DK)
(72) Erfinder: Brøndlund, Marianne, 8462 Harlev (DK)
(74) Vertreter: Laufhütte, Dieter

(57) **Zusammenfassung**

Die vorliegende Erfindung betrifft ein Stoffgemisch, wobei das Stoffgemisch Ribose, Alanin, Nikotinsäure und Ascorbinsäure enthält.

## Beschreibung

Die Erfindung betrifft ein Stoffgemisch, die Verwendung eines Stoffgemisches zur Herstellung eines Medikamentes bzw. eines Nahrungsergänzungsmittels, sowie eine Infusionslösung.

Ziel der vorliegenden Erfindung ist es, ein Arzneimittel bzw. Nahrungsergänzungsmittel mit natürlichen Inhaltsstoffen schaffen, welches in der Behandlung einer Vielzahl von Krankheiten eingesetzt werden kann.

Dies wird durch ein Stoffgemisch nach Anspruch 1, durch eine Infusionslösung gemäß Anspruch 8 sowie durch eine Verwendung eines Stoffgemisches nach Anspruch 10 erreicht. Vorteilhafte Ausführungsformen ergeben sich aus den Unteransprüchen.

Demgemäß betrifft die Erfindung ein Stoffgemisch enthaltend Ribose, Alanin, Nikotinsäure und Ascorbinsäure. Das erfindungsgemäße Stoffgemisch dient zur Bereitstellung eines Arzneimittels und/oder Nahrungsergänzungsmittels, welches vorzugsweise ausschließlich natürliche Inhaltsstoffe aufweist.

In einer Ausführungsform ist die verwendete Ribose D-Ribose. Diese stellt einen wichtigen Baustein im menschlichen Körper, beispielsweise in der RNA-Backbone und als Bestandteil des Adenosins, und damit des ATP, ADP, AMP oder cAMP.

In einer Ausführungsform ist das verwendete Alanin razemisches (DL)-α-Alanin. Alanin kann als Bestandteil von Infusionslösungen zur parenteralen Ernährung dienen.

In einer Ausführungsform ist die verwendete Ascorbinsäure L-Ascorbinsäure, die auch unter dem Trivialnamen Vitamin C bekannt ist und die Abwehrkräfte des Körpers stärkt.

In einer Ausführungsform enthält das erfindungsgemäße Stoffgemisch zusätzlich Thiamin. Thiamin ist auch unter dem Trivialnamen Vitamin B1 bekannt und stärkt die Abwehrkräfte und das Nervensystem der Patienten.

Nikotinsäure ist auch unter dem Trivialnamen Vitamin B5 bekannt und spielt eine wichtige Rolle im Stoffwechsel.

Ein erfindungsgemäßes Stoffgemisch stellt in dieser Zusammensetzung also eine neue Kombination von Wirkstoffen dar, die verschiedene Körperfunktionen stärkt und bei geeigneter Verabreichung und Dosierung erfolgreich zur Behandlung und zur unterstützenden Behandlung einer Vielzahl von Krankheiten dienen kann.

In einer Ausführungsform handelt es sich bei dem erfindungsgemäßen Stoffgemisch um ein festes Stoffgemisch, das die Bestandteile als Feststoffe enthält. Ribose, Alanin, Nikotinsäure und Ascorbinsäure, sowie das gegebenenfalls vorhandene Thiamin liegen bei Raumtemperatur als Feststoff, typischerweise als weißes Pulver vor.

In einer Ausführungsform ist das erfindungsgemäße, feste Stoffgemisch ein pulverförmiges Gemisch der pulverförmigen Bestandteile Ribose, Alanin, Nikotinsäure und Ascorbinsäure, sowie gegebenenfalls Thiamin. In einer Ausführungsform besteht das erfindungsgemäße, feste Stoffgemisch im Wesentlichen oder ausschließlich aus diesen Bestandteilen.

In einer Ausführungsform wird das erfindungsgemäße, feste Stoffgemisch in einer bestimmten Dosierung in separaten Behältnissen bereitgestellt. Geeignete Behältnisse umfassen beispielsweise Kapseln, Papiertaschen, Papierbriefchen, Plastikhülsen, oder Plastikröhrchen. Da das erfindungsgemäße, feste Stoffgemisch lichtempfindlich ist, empfiehlt es sich zudem, dass das Behältnis das Stoffgemisch vor Lichteinfall von außen schützt. Besonders geeignete Behältnisse umfassen daher auch solche aus getöntem Glas.

In einer Ausführungsform enthält ein Behältnis genau eine Dosierungseinheit, die zwischen etwa 40 g und etwa 250 g Ribose, zwischen etwa 5 g und etwa 50 g Alanin, zwischen etwa 3 g und etwa 30 g Nikotinsäure, und zwischen etwa 5 g und etwa 75 g Ascorbinsäure enthält. Dies stellt eine geeignete Dosierung hinsichtlich der Stoffmengen und insbesondere der Stoffmengenverhältnisse bei einer weiteren Verwendung als bzw. zur Herstellung von einem Medikament und/oder Nahrungsergänzungsmittel, beispielsweise durch Auflösen und gegebenenfalls Verdünnen, dar.

Eine bevorzugte Menge an Ribose innerhalb einer Dosierungseinheit liegt zwischen etwa 60 g und etwa 200 g. Eine bevorzugte Menge an Alanin innerhalb einer Dosierungseinheit liegt zwischen etwa 10 g und etwa 40 g. Eine bevorzugte Menge an Nikotinsäure innerhalb einer Dosierungseinheit liegt zwischen etwa 5 g und etwa 25 g. Eine bevorzugte Menge an Ascorbinsäure innerhalb einer Dosierungseinheit liegt zwischen etwa 10 g und etwa 50 g.

Sollte das erfindungsgemäße, feste Stoffgemisch zusätzlich Thiamin enthalten, kann eine Dosierungseinheit in einer Ausführungsform zwischen etwa 2 g und etwa 8 g, vorzugsweise zwischen etwa 4 g und etwa 6 g Thiamin enthalten.

In einer Ausführungsform enthält eine Dosierungseinheit eines erfindungsgemäßen Stoffgemisches zwischen etwa 65 g und etwa 80 g, vorzugsweise zwischen etwa 70 g und etwa 74 g Ribose, zwischen etwa 15 g und etwa 25 g, vorzugsweise zwischen etwa 17 g und etwa 21 g Alanin, zwischen etwa 10 g und etwa 15 g, vorzugsweise zwischen etwa 11 g und etwa 13 g Nikotinsäure sowie zwischen etwa 30 g und etwa 40 g, vorzugsweise zwischen etwa 34 g und etwa 38 g Ascorbinsäure. Ein solches Stoffgemisch eignet sich insbesondere zur Bereitstellung eines Medikaments und/oder Nahrungsergänzungsmittels zur Behandlung bzw. unterstützenden Behandlung verschiedener Krebsarten, von lymphogener Leukämie, von Lupus erythematodes, oder Diabetes mellitus. Dieses erfindungsgemäße, feste Stoffgemisch wird im weiteren Verlauf als festes Stoffgemisch A bezeichnet.

In einer anderen Ausführungsform enthält eine Dosierungseinheit eines erfindungsgemäßen Stoffgemisches zwischen etwa 55 g und etwa 65 g, vorzugsweise zwischen etwa 58 g und etwa 62 g Ribose, zwischen etwa 5 g und etwa 15 g, vorzugsweise zwischen etwa 8 g und etwa 12 g Alanin, zwischen etwa 12 g und etwa 18 g, vorzugsweise zwischen etwa 13 g und etwa 17 g Nikotinsäure, sowie zwischen etwa 5 g und etwa 15 g, vorzugsweise zwischen etwa 8 g und etwa 12 g Ascorbinsäure. Ein solches Stoffgemisch eignet sich insbesondere zur Bereitstellung eines Medikaments und/oder Nahrungsergänzungsmittels zur Behandlung bzw. unterstützenden Behandlung von koronarer Herzkrankheit, Herzinsuffizienten und Fettstoffwechselstörungen. Dieses erfindungsgemäße, feste Stoffgemisch wird im weiteren Verlauf als festes Stoffgemisch B bezeichnet.

In einer wiederum anderen Ausführungsform enthält eine Dosierungseinheit eines erfindungsgemäßen Stoffgemisches zwischen etwa 55 g und etwa 65 g, vorzugsweise zwischen etwa 58 g und etwa 62 g Ribose, zwischen etwa 15 g und etwa 25 g, vorzugsweise zwischen etwa 17 g und etwa 21 g Alanin, zwischen etwa 8 g und etwa 15 g, vorzugsweise zwischen etwa 9 g und etwa 13 g Nikotinsäure, zwischen etwa 40 g und etwa 55 g, vorzugsweise zwischen etwa 46 g und etwa 50 g Ascorbinsäure sowie zwischen etwa 3 g und etwa 7 g, vorzugsweise zwischen etwa 4 g und etwa 6 g Thiamin. Ein solches Stoffgemisch eignet sich insbesondere zur Bereitstellung eines Medikaments und/oder Nahrungsergänzungsmittels zur Behandlung bzw. unterstützenden Behandlung von degenerativen Knochenerkrankungen, rheumatodier Arthritis sowie von Knochenmetastasen von Tumoren. Dieses erfindungsgemäße, feste Stoffgemisch wird im weiteren Verlauf als festes Stoffgemisch C bezeichnet.

In einer Ausführungsform handelt es sich bei dem erfindungsgemäßen Stoffgemisch um ein flüssiges Stoffgemisch, das die Bestandteile in wässriger Lösung enthält. Ribose, Alanin, Nikotinsäure und Ascorbinsäure, sowie das gegebenenfalls vorhandene Thiamin sind gut wasserlöslich, wodurch diese problemlos bei Raumtemperatur in reinem Wasser oder wässrigen Lösungen aufgelöst werden können.

Da das erfindungsgemäße, flüssige Stoffgemisch lichtempfindlich ist, empfiehlt es sich das Stoffgemisch vor Lichteinfall von außen zu schützen. Besonders geeignete Behältnisse umfassen beispielsweise Ampullen aus getöntem Glas.

In einer Ausführungsform enthält ein erfindungsgemäßes, flüssiges Stoffgemisch die Bestandteile in Lösung einer physiologischen Kochsalzlösung (auch isotonische Kochsalzlösung oder 0,9%ige Kochsalzlösung). Auch der Einsatz anderer isotonischer Lösungen ist denkbar.

In einer Ausführungsform kann vorgesehen sein, dass ein erfindungsgemäßes flüssiges Stoffgemisch im Wesentlichen oder ausschließlich aus den Bestandteilen Ribose, Alanin, Nikotinsäure, Ascorbinsäure, gegebenenfalls Thiamin, und Wasser oder physiologischer Kochsalzlösung besteht.

In einer bevorzugten Ausführungsform enthält ein erfindungsgemäßes, flüssiges Stoffgemisch zwischen etwa 40 g/l und etwa 250 g/l Ribose, zwischen etwa 5 g/l und etwa 50 g/l Alanin, zwischen etwa 3 g/l und etwa 30 g/l Nikotinsäure, und zwischen etwa 5 g/l und etwa 75 g/l Ascorbinsäure. Dies stellt eine geeignete Dosierung hinsichtlich der Konzentrationen und insbesondere der Konzentrationsverhältnisse bei einer Verwendung als Medikament und/oder Nahrungsergänzungsmittels oder bei einer weiteren Verwendung zur Herstellung eines Medikaments und/oder Nahrungsergänzungsmittels durch beispielsweise Verdünnen dar.

Ein bevorzugter Konzentrationsbereich für Ribose liegt zwischen etwa 60 g/l und etwa 200 g/l. Ein bevorzugter Konzentrationsbereich für Alanin liegt zwischen etwa 10 g/l und etwa 40 g/l. Ein bevorzugter Konzentrationsbereich für Nikotinsäure liegt zwischen etwa 5 g/l und etwa 25 g/l. Ein bevorzugter Konzentrationsbereich für Ascorbinsäure liegt zwischen etwa 10 g/l und etwa 50 g/l.

Sollte das erfindungsgemäße, flüssige Stoffgemisch zusätzlich Thiamin enthalten, beträgt dessen Konzentration in einer Ausführungsform zwischen etwa 2 g/l und etwa 8 g/l, und vorzugsweise zwischen etwa 4 g/l und etwa 6 g/l.

In einer Ausführungsform enthält ein erfindungsgemäßes, flüssiges Stoffgemisch zwischen etwa 65 g/l und etwa 80 g/l, vorzugsweise zwischen etwa 70 g/l und etwa 74 g/l Ribose, zwischen etwa 15 g/l und etwa 25 g/l, vorzugsweise zwischen etwa 17 g/l und etwa 21 g/l Alanin, zwischen etwa 10 g/l und etwa 15 g/l, vorzugsweise zwischen etwa 11 g/l und etwa 13 g/l Nikotinsäure sowie zwischen etwa 30 g/l und etwa 40 g/l, vorzugsweise zwischen etwa 34 g/l und etwa 38 g/l Ascorbinsäure. Ein solches Stoffgemisch eignet sich insbesondere zur Behandlung bzw. zur Bereitstellung eines Medikaments und/oder Nahrungsergänzungsmittels, insbesondere einer Infusionslösung zur Behandlung bzw. unterstützenden Behandlung verschiedener Krebsarten, von lymphogener Leukämie, von Lupus erythematodes, oder Diabetes mellitus. Dieses erfindungsgemäße, flüssige Stoffgemisch wird im weiteren Verlauf als flüssiges Stoffgemisch A bezeichnet.

In einer Ausführungsform enthält ein erfindungsgemäßes, flüssiges Stoffgemisch zwischen etwa 55 g/l und etwa 65 g/l, vorzugsweise zwischen etwa 58 g/l und etwa 62 g/l Ribose, zwischen etwa 5 g/l und etwa 15 g/l, vorzugsweise zwischen etwa 8 g/l und etwa 12 g/l Alanin, zwischen etwa 12 g/l und etwa 18 g/l, vorzugsweise zwischen etwa 13 g/l und etwa 17 g/l Nikotinsäure, sowie zwischen etwa 5 g/l und etwa 15 g/l, vorzugsweise zwischen etwa 8 g/l und etwa 12 g/l Ascorbinsäure. Ein solches Stoffgemisch eignet sich insbesondere zur Behandlung bzw. zur Bereitstellung eines Medikaments und/oder Nahrungsergänzungsmittels, insbesondere einer Infusionslösung zur Behandlung bzw. unterstützenden Behandlung von koronarer Herzkrankheit, Herzinsuffizienten und Fettstoffwechselstörungen. Dieses erfindungsgemäße, flüssige Stoffgemisch wird im weiteren Verlauf als flüssiges Stoffgemisch B bezeichnet.

In einer Ausführungsform enthält ein erfindungsgemäßes, flüssiges Stoffgemisch zwischen etwa 55 g/l und etwa 65 g/l, vorzugsweise zwischen etwa 58 g/l und etwa 62 g/l Ribose, zwischen etwa 15 g/l und etwa 25 g/l, vorzugsweise zwischen etwa 17 g/l und etwa 21 g/l Alanin, zwischen etwa 8 g/l und etwa 15 g/l, vorzugsweise zwischen etwa 9 g/l und etwa 13 g/l Nikotinsäure, zwischen etwa 40 g/l und etwa 55 g/1, vorzugsweise zwischen etwa 46 g/l und etwa 50 g/l Ascorbinsäure sowie zwischen etwa 3 g/l und etwa 7 g/l, vorzugsweise zwischen etwa 4 g/l und etwa 6 g/l Thiamin. Ein solches Stoffgemisch eignet sich insbesondere zur Behandlung bzw. zur Bereitstellung eines Medikaments und/oder Nahrungsergänzungsmittels, insbesondere einer Infusionslösung zur Behandlung bzw. unterstützenden Behandlung von degenerativen Knochenerkrankungen, rheumatodier Arthritis sowie von Knochenmetastasen von Tumoren. Dieses erfindungsgemäße, flüssige Stoffgemisch wird im weiteren Verlauf als flüssiges Stoffgemisch C bezeichnet.

In einer Ausführungsform handelt es sich bei dem erfindungsgemäßen, flüssigen Stoffgemisch um ein Konzentrat. Dies kann zum Erhalt eines verabreichungsfertigen Medikaments und/oder Nahrungsergänzungsmittels beispielsweise weiter mit Wasser oder physiologischer Kochsalzlösung verdünnt werden. Die oben genannten Mischungsverhältnisse im Konzentrat sind dabei bereits so gewählt, dass durch eine bloße Verdünnung die gewünschten Stoffmengenverhältnisse erreicht werden.

In einer anderen Ausführungsform handelt es sich bei dem erfindungsgemäßen, flüssigen Stoffgemisch um ein verabreichungsfertiges Medikament und/oder Nahrungsergänzungsmittel. Dies kann zur Behandlung eines Patienten in geeigneter Dosierung beispielsweise intravenös verabreicht werden. Geeignete Dosierungen umfassen zwischen etwa 5 ml und etwa 15 ml, vorzugsweise zwischen etwa 8 ml und etwa 12 ml. Besonders bevorzugt ist die intravenöse Verabreichung von genau 10 ml des erfindungsgemäßen, flüssigen Stoffgemisches.

Zusätzlich zur Verabreichung des erfindungsgemäßen, flüssigen Stoffgemischs kann die vorgelagerte oder nachgelagerte, vorzugsweise intravenöse Verabreichung eines homöopathischen Wirkstoffs die Wirkung und die Heilkraft eines erfindungsgemäßen Stoffgemisches begründen oder verstärken.

Bevorzugte homöopathische Wirkstoffe für diese Anwendung umfassen Wirkstoffe ausgewählt aus der Gruppe Magnesium phosphoricum, Natrium pyruvicum, Natrium diethyloxalaceticum, Acidum citricum, Acidum cis-aconiticum, Barium oxalsuccinicum, Acidum α-ketoglutaricum, Acidum succinicum, Acidum fumaricum, Acidum DL-malicum, Strychnos nux-vomica, Ubidecarenonum, Natrium pyruvicum, Strychnos ignatii, Hepar suis, Silybum marianum, Ren suis, Calendula officinalis, Atropa bella-donna, Acontium napellus, Bellis perennis, Hypericum perforatum, Echinacea, Echinacea purpurea, Symphytum officinale, Matricaria recutita, Achillea millefolium, Mercurius solubilis Hahnemanni, Hepar sulfuris, Hamamelis virginiana, Arnica montana und Zincum valerianicum.

Die Erfindung betrifft ferner ein Verfahren zur Bereitstellung eines erfindungsgemäßen, flüssigen Stoffgemisches, wobei ein erfindungsgemäßes, festes Stoffgemisch in Wasser, einer physiologischen Kochsalzlösung oder einer anderen physiologisch verträglichen wässrigen Lösung aufgelöst wird.

In einer Ausführungsform erfolgt dieser Lösevorgang bei Raumtemperatur (ca. 21°C) und/oder unter vorsichtigem Rühren und/oder unter sterilen Bedingungen.

In einer Ausführungsform wird eine Dosierungseinheit eines erfindungsgemäßen festen Stoffgemisches in zwischen etwa 500 ml und etwa 1500 ml, vorzugsweise in zwischen etwa 800 ml und etwa 1200 ml und weiter vorzugsweise in ungefähr oder genau 1000 ml Wasser, physiologischer Kochsalzlösung oder einer anderen physiologisch verträglichen wässrigen Lösung aufgelöst.

In einer Ausführungsform werden der Lösung vor und/oder während und/oder nach dem Lösevorgang ein oder mehrere homöopathische Wirkstoffe zugesetzt.

Die Erfindung betrifft des Weiteren eine Infusionslösung, die die Inhaltsstoffe eines erfindungsgemäßen, flüssigen Stoffgemisches in etwa 20-facher bis etwa 60-facher Verdünnung enthält. Vorzugsweise enthält eine erfindungsgemäße Infusionslösung diese Inhaltsstoffe in 25-facher oder etwa 50-facher Verdünnung. Als Basis für die Infusionslösung dient vorzugsweise eine physiologische Kochsalzlösung (auch isotonische Kochsalzlösung oder 0,9%ige Kochsalzlösung). Auch der Einsatz anderer Infusionslösungen ist denkbar. Die Infusionen eigenen sich zur parenteralen Verabreichung an einen Patienten.

Je nach der gewünschten Behandlungsintensität sowie dem Zustand des Patienten kann ein behandelnder Arzt den Verdünnungsgrad der Infusion innerhalb der erfinderischen Schranken anpassen.

In einer Ausführungsform enthält eine erfindungsgemäße Infusion, die die Inhaltsstoffe eines erfindungsgemäßen, flüssigen Stoffgemisches in 25-facher Verdünnung enthält, zwischen etwa 1,5 g/l und etwa 10 g/l Ribose, zwischen etwa 0,2 g/l und etwa 2 g/l Alanin, zwischen etwa 0,10 g/l und etwa 1 g/l Nikotinsäure, und zwischen etwa 0,2 g/l und etwa 3 g/l Ascorbinsäure, und eine erfindungsgemäße Infusion, die die Inhaltsstoffe eines erfindungsgemäßen, flüssigen Stoffgemisches in 50-facher Verdünnung enthält, zwischen etwa 0,7 g/l und etwa 5 g/l Ribose, zwischen etwa 0,1 g/l und etwa 1 g/l Alanin, zwischen etwa 0,05 g/l und etwa 0,5 g/l Nikotinsäure, und zwischen etwa 0,1 g/l und etwa 1,5 g/l Ascorbinsäure. Dies stellt ein geeignetes Konzentrationsverhältnis bei einer Behandlung dar.

Ein bevorzugter Konzentrationsbereich für Ribose liegt bei 25-facher Verdünnung zwischen etwa 2,5 g/l und etwa 8 g/l und bei 50-facher Verdünnung zwischen etwa 1,2 g/l und etwa 4 g/l. Ein bevorzugter Konzentrationsbereich für Alanin liegt bei 25-facher Verdünnung zwischen etwa 0,5 g/l und etwa 1,5 g/l und bei 50-facher Verdünnung zwischen etwa 0,2 g/l und etwa 0,8 g/l. Ein bevorzugter Konzentrationsbereich für Nikotinsäure liegt bei 25-facher Verdünnung zwischen etwa 0,2 g/l und etwa 1 g/l und bei 50-facher Verdünnung zwischen etwa 0,1 g/l und etwa 0,5 g/l. Ein bevorzugter Konzentrationsbereich für Ascorbinsäure liegt bei 25-facher Verdünnung zwischen etwa 0,4 g/l und etwa 2 g/l und bei 50-facher Verdünnung zwischen etwa 0,2 g/l und etwa 1 g/l.

Sollte die erfindungsgemäße Infusion zusätzlich Thiamin enthalten, beträgt dessen Konzentration in einer Ausführungsform bei 25-facher Verdünnung zwischen etwa 0,1 g/l und etwa 0,3 g/l, und vorzugsweise zwischen etwa 0,15 g/l und etwa 0,25 g/l und bei 50-facher Verdünnung zwischen etwa 0,05 g/l und etwa 0,2 g/l.

In einer Ausführungsform enthält eine erfindungsgemäße Infusion bei 25-facher Verdünnung zwischen etwa 2,6 g/l und etwa 3,2 g/l, vorzugsweise zwischen etwa 2,8 g/l und etwa 3 g/l Ribose, zwischen etwa 0,6 g/l und etwa 1 g/l, vorzugsweise zwischen etwa 0,7 g/l und etwa 0,9 g/l Alanin, zwischen etwa 0,4 g/l und etwa 0,6 g/l, vorzugsweise etwa 0,5 g/l Nikotinsäure sowie zwischen etwa 1,2 g/l und etwa 1,6 g/l, vorzugsweise zwischen etwa 1,3 g/l und etwa 1,5 g/l Ascorbinsäure, und bei 50-facher Verdünnung zwischen etwa 1,3 g/l und etwa 1,6 g/l, vorzugsweise zwischen etwa 1,4 g/l und etwa 1,5 g/l Ribose, zwischen etwa 0,3 g/l und etwa 0,5 g/l Alanin, zwischen etwa 0,2 g/l und etwa 0,3 g/l Nikotinsäure sowie zwischen etwa 0,6 g/l und etwa 0,8 g/l Ascorbinsäure. Eine solche Infusion eignet sich insbesondere zur Behandlung bzw. unterstützenden Behandlung verschiedener Krebsarten, von lymphogener Leukämie, von Lupus erythematodes, oder Diabetes mellitus. Diese erfindungsgemäße Infusion wird im weiteren Verlauf als Infusion A bezeichnet.

In einer Ausführungsform enthält eine erfindungsgemäße Infusion bei 25-facher Verdünnung zwischen etwa 2,2 g/l und etwa 2,6 g/l, vorzugsweise zwischen etwa 2,3 g/l und etwa 2,5 g/l Ribose, zwischen etwa 0,2 g/l und etwa 0,6 g/l, vorzugsweise zwischen etwa 0,3 g/l und etwa 0,5 g/l Alanin, zwischen etwa 0,5 g/l und etwa 0,7 g/l, vorzugsweise etwa 0,6 g/l Nikotinsäure, sowie zwischen etwa 0,2 g/l und etwa 0,6 g/l, vorzugsweise zwischen etwa 0,3 g/l und etwa 0,5 g/l Ascorbinsäure, und bei 50-facher Verdünnung zwischen etwa 1,1 g/l und etwa 1,3 g/l Ribose, zwischen etwa 0,1 g/l und etwa 0,3 g/l Alanin, zwischen etwa 0,2 g/l und etwa 0,4 g/l Nikotinsäure, sowie zwischen etwa 0,1 g/l und etwa 0,3 g/l Ascorbinsäure. Eine solche Infusion eignet sich insbesondere zur Behandlung bzw. unterstützenden Behandlung von koronarer Herzkrankheit, Herzinsuffizienten und Fettstoffwechselstörungen. Diese erfindungsgemäße Infusion wird im weiteren Verlauf als Infusion B bezeichnet.

In einer Ausführungsform enthält eine erfindungsgemäße Infusion bei 25-facher Verdünnung zwischen etwa 2,2 g/l und etwa 2,6 g/l, vorzugsweise zwischen etwa 2,3 g/l und etwa 2,5 g/l Ribose, zwischen etwa 0,6 g/l und etwa 1 g/l, vorzugsweise zwischen etwa 0,7 g/l und etwa 0,9 g/l Alanin, zwischen etwa 0,3 g/l und etwa 0,6 g/l, vorzugsweise zwischen etwa 0,4 g/l und etwa 0,5 g/l Nikotinsäure, zwischen etwa 1,6 g/l und etwa 2,2 g/l, vorzugsweise zwischen etwa 1,8 g/l und etwa 2 g/l Ascorbinsäure sowie zwischen etwa 0,1 g/l und etwa 0,3 g/l, vorzugsweise etwa 0,2 g/l Thiamin, und bei 50-facher Verdünnung zwischen etwa 1,1 g/l und etwa 1,3 g/l Ribose, zwischen etwa 0,3 g/l und etwa 0,5 g/l Alanin, zwischen etwa 0,1 g/l und etwa 0,3 g/l Nikotinsäure, zwischen etwa 0,8 g/l und etwa 1,1 g/l, vorzugsweise zwischen etwa 0,9 g/l und etwa 1 g/l Ascorbinsäure sowie zwischen etwa 0,05 g/l und etwa 0,15 g/l Thiamin. Eine solche Infusion eignet sich insbesondere zur Behandlung bzw. unterstützenden Behandlung von degenerativen Knochenerkrankungen, rheumatodier Arthritis sowie von Knochenmetastasen von Tumoren. Diese erfindungsgemäße Infusion wird im weiteren Verlauf als Infusion C bezeichnet.

In einer Ausführungsform enthält eine erfindungsgemäße Infusion zumindest einen homöopathischen Wirkstoff. Bevorzugte homöopathische Wirkstoffe umfassen dabei Wirkstoffe ausgewählt aus der Gruppe Magnesium phosphoricum, Natrium pyruvicum, Natrium diethyloxalaceticum, Acidum citricum, Acidum cis-aconiticum, Barium oxalsuccinicum, Acidum α-ketoglutaricum, Acidum succinicum, Acidum fumaricum, Acidum DL-malicum, Strychnos nux-vomica, Ubidecarenonum, Natrium pyruvicum, Strychnos ignatii, Hepar suis, Silybum marianum, Ren suis, Calendula officinalis, Atropa bella-donna, Acontium napellus, Bellis perennis, Hypericum perforatum, Echinacea, Echinacea purpurea, Symphytum officinale, Matricaria recutita, Achillea millefolium, Mercurius solubilis Hahnemanni, Hepar sulfuris, Hamamelis virginiana, Arnica montana und Zincum valerianicum.

Dieser Zusatz der homöopathischen Wirkstoffe kann die Wirkung und die Heilkraft einer erfindungsgemäßen Infusion begründen oder verstärken.

Die Erfindung betrifft des Weiteren ein Verfahren zur Bereitstellung einer erfindungsgemäßen Infusionslösung durch Verdünnung eines erfindungsgemäßen, flüssigen Stoffgemisches in einer Basislösung wie vorzugsweise physiologischer Kochsalzlösung.

In einer Ausführungsform werden etwa 10 ml (zwischen etwa 8 ml und etwa 12 ml) eines erfindungsgemäßen flüssigen Stoffgemisches in entweder etwa 250 ml oder etwa 500 ml einer Infusionsbasislösung verdünnt. Als Infusionsbasislösung dient vorzugsweise eine physiologische Kochsalzlösung (auch isotonische Kochsalzlösung oder 0,9%ige Kochsalzlösung). Auch der Einsatz anderer Infusionslösungen ist denkbar.

In einer Ausführungsform werden der Infusion vor und/oder während und/oder nach dem Verdünnungsvorgang ein oder mehrere homöopathische Wirkstoffe zugesetzt. Die Erfindung betrifft ferner die Verwendung eines erfindungsgemäßen Stoffgemisches zur Herstellung eines Medikaments und/oder Nahrungsergänzungsmittels, vorzugsweise einer Infusionslösung, und weiter vorzugsweise einer erfindungsgemäßen Infusionslösung.

Ein derartiges Medikament und/oder Nahrungsergänzungsmittel bzw. eine erfindungsgemäße Infusionslösung können bei der alleinigen Behandlung oder als Ergänzung bei der schulmedizinischen Behandlung eingesetzt werden. Die Verabreichung stärkt den Körper und das Immunsystem, hebt das Wohlbefinden des Patienten und begünstigt so die Heilung.

Bevorzugt ist die Verwendung eines erfindungsgemäßen Stoffgemisches zur Herstellung eines Medikamentes und/oder Nahrungsergänzungsmittels, vorzugsweise einer Infusion, zur Behandlung von Erkrankungen ausgewählt aus der Gruppe Krebs, Knochenmetastasen, lymohogene Leukämie, Lupus erythematodes, Diabetes mellitus, koronarer Herzkrankheiten, Herzinsuffizienz, Fettstoffwechselstörungen, degenerativen Knochenerkrankungen und rheumatioder Arthritis.

Eine Ausführungsform betrifft die Verwendung eines erfindungsgemäßen Stoffgemisches A zur Herstellung einer erfindungsgemäßen Infusion A zur Behandlung bzw. unterstützenden Behandlung verschiedener Krebsarten, von lymphogener Leukämie, von Lupus erythematodes, oder Diabetes mellitus.

Eine Ausführungsform betrifft die Verwendung eines erfindungsgemäßen Stoffgemisches B zur Herstellung einer erfindungsgemäßen Infusion B zur Behandlung bzw. unterstützenden Behandlung von koronarer Herzkrankheit, Herzinsuffizienten und Fettstoffwechselstörungen.

Eine Ausführungsform betrifft die Verwendung eines erfindungsgemäßen Stoffgemisches C zur Herstellung einer erfindungsgemäßen Infusion C zur Behandlung bzw. unterstützenden Behandlung von degenerativen Knochenerkrankungen, rheumatodier Arthritis sowie von Knochenmetastasen von Tumoren.

Weitere Einzelheiten und Vorteile der Erfindung ergeben sich aus den folgenden Ausführungsbeispielen.

### Beispiel 1:

In einer Verpackungseinheit, beispielsweise einem beschichteten Papiersäckchen, werden 72 g D-Ribose, 19,2 g DL-α-Alanin, 12 g Nikotinsäure und 36 g L-Ascorbinsäure in Pulverform bereitgestellt.

Diese werden in 1 l 0,9%-iger Kochsalzlösung bei 21°C unter vorsichtigem Rühren und unter sterilen Bedingungen aufgelöst. Dabei entsteht ein Konzentrat mit 72 g/l D-Ribose, 19,2 g/l DL-α-Alanin, 12 g/l Nikotinsäure, 36 g/l L-Ascorbinsäure und 9 g/l NaCl.

### Beispiel 2:

In einer Verpackungseinheit, beispielsweise einem beschichteten Papiersäckchen, werden 60 g D-Ribose, 10,3 g DL-α-Alanin, 15 g Nikotinsäure sowie 10 g L-Ascorbinsäure in Pulverform bereitgestellt.

Diese werden wie in Beispiel 1 dargestellt in 1 l isotonischer Kochsalzlösung aufgelöst, wobei ein Konzentrat mit 60 g/l D-Ribose, 10,3 g/l DL-α-Alanin, 15 g/l Nikotinsäure, 10 g/l L-Ascorbinsäure und 9 g/l NaCl erhalten wird.

### Beispiel 3:

In einer Verpackungseinheit, beispielsweise einem beschichteten Papiersäckchen, werden 60 g D-Ribose, 19,2 g DL-α-Alanin, 10,8 g Nikotinsäure, 48 g L-Ascorbinsäure und 4,8 g in Pulverform Thiamin bereitgestellt.

Diese werden wie in Beispiel 1 dargestellt in 1 l isotonischer Kochsalzlösung aufgelöst, wobei ein Konzentrat mit 60 g/l D-Ribose, 19,2 g/l DL-α-Alanin, 10,8 g/l Nikotinsäure, 48 g/l L-Ascorbinsäure, 4,8 g/l Thiamin und 9 g/l NaCl erhalten wird.

### Beispiel 4:

Es wird ein homöopathisches Wirkstoffgemisch bereitgestellt, wobei der Inhalt folgender Ampullen der Firma Heel - Healthcare Designed by Nature in einem Glasbehälter vereinigt werden:
10 Ampullen zu 1,1 ml Zitronensäurezyklus-Heel,
10 Ampullen zu 1,1 ml Nux vomica-Injeel S,
5 Ampullen zu 1,1 ml Ubichinon-Injeel forte,
5 Ampullen zu 1,1 ml Acidum DL-malicum-Injeel,
5 Ampullen zu 1,1 ml Natrium pyruvicum-Injeel,
10 Ampullen zu 1,1 ml Ignatia-Injeel S,
10 Ampullen zu 1,1 ml Hepar suis-Injeel,
10 Ampullen zu 1,1 ml Carduus marianus-Injeel forte,
10 Ampullen zu 1,1 ml Ren suis-Injeel,
5 Ampullen zu 5 ml Traumeel S,
5 Ampullen zu 5 ml Belladonna-Homaccord, und
10 Ampullen zu 1,1 ml Zincum valerianicum-Injeel.

### Beispiel 5:

Das Konzentrat aus Beispiel 1 wird in Ampullen aus getöntem Glas zu jeweils 10 ml abgefüllt. Der Inhalt einer Ampulle wird als ergänzende Therapie bei der Behandlung von Krebs, lymohogener Leukämie, Lupus erythematodes sowie von Diabetes mellitus intravenös an den Patienten verabreicht.

### Beispiel 6:

Zusätzlich zur in Beispiel 5 beschriebenen Therapie werden nachgelagert 10 ml des homöopathischen Wirkstoffgemisches aus Beispiel 4 intravenös verabreicht, um die Heilkraft des erfindungsgemäßen Stoffgemisches aus Beispiel 1 zu verstärken.

### Beispiel 7:

10 ml des Konzentrats aus Beispiel 1 werden zu 250 ml oder 500 ml einer Infusionslösung, vorzugsweise 0,9%-iger Kochsalzlösung zugesetzt. Der Infusionslösung werden ferner 10 ml des homöopathischen Wirkstoffgemischs aus Beispiel 4 zugesetzt.

Die entstehende Infusionslösung wird als ergänzende Therapie bei der Behandlung von Krebs, lymohogener Leukämie, Lupus erythematodes sowie von Diabetes mellitus intravenös an den Patienten verabreicht.

### Beispiel 8:

Das Konzentrat aus Beispiel 2 wird in Ampullen aus getöntem Glas zu jeweils 10 ml abgefüllt. Der Inhalt einer Ampulle wird als ergänzende Therapie bei der Behandlung von koronaren Herzkrankheiten, Herzinsuffizienz sowie von Fettstoffwechselstörungen intravenös an den Patienten verabreicht.

### Beispiel 9:

Zusätzlich zur in Beispiel 8 beschriebenen Therapie werden nachgelagert 10 ml des homöopathischen Wirkstoffgemisches aus Beispiel 4 intravenös verabreicht, um die Heilkraft des erfindungsgemäßen Stoffgemisches aus Beispiel 2 zu verstärken.

### Beispiel 10:

10 ml des Konzentrats aus Beispiel 2 werden zu 250 ml oder 500 ml einer Infusionslösung, vorzugsweise 0,9%-iger Kochsalzlösung zugesetzt. Der Infusionslösung werden ferner 10 ml des homöopathischen Wirkstoffgemischs aus Beispiel 4 zugesetzt.

Die entstehende Infusionslösung wird als ergänzende Therapie bei der Behandlung von koronaren Herzkrankheiten, Herzinsuffizienz sowie von Fettstoffwechseistörungen intravenös an den Patienten verabreicht.

### Beispiel 11:

Das Konzentrat aus Beispiel 3 wird in Ampullen aus getöntem Glas zu jeweils 10 ml abgefüllt. Der Inhalt einer Ampulle wird als ergänzende Therapie bei der Behandlung von degenerativen Knochenerkrankungen, rheumatoider Arthritis sowie Knochenmetastasen intravenös an den Patienten verabreicht.

### Beispiel 12:

Zusätzlich zur in Beispiel 11 beschriebenen Therapie werden nachgelagert 10 ml des homöopathischen Wirkstoffgemisches aus Beispiel 4 intravenös verabreicht, um die Heilkraft des erfindungsgemäßen Stoffgemisches aus Beispiel 3 zu verstärken.

### Beispiel 13:

10 ml des Konzentrats aus Beispiel 3 werden zu 250 ml oder 500 ml einer Infusionslösung, vorzugsweise 0,9%-iger Kochsalzlösung zugesetzt. Der Infusionslösung werden ferner 10 ml des homöopathischen Wirkstoffgemischs aus Beispiel 4 zugesetzt.

Die entstehende Infusionslösung wird als ergänzende Therapie bei der Behandlung von degenerativen Knochenerkrankungen, rheumatoider Arthritis sowie Knochenmetastasen intravenös an den Patienten verabreicht.

## Patentansprüche

1. Stoffgemisch, **dadurch gekennzeichnet, dass** das Stoffgemisch Ribose, Alanin, Nikotinsäure und Ascorbinsäure enthält.

2. Stoffgemisch nach Anspruch 1, wobei es sich bei der Ribose um D-Ribose und/oder wobei es sich bei dem Alanin um DL-α-Alanin und/oder wobei es sich bei der Ascorbinsäure um L-Ascorbinsäure handelt.

3. Stoffgemisch nach Anspruch 1 oder 2, wobei das Stoffgemisch zusätzlich Thiamin enthält.

4. Stoffgemisch nach einem der vorangehenden Ansprüche, wobei es sich um ein festes Stoffgemisch handelt, das Ribose, Alanin, Nikotinsäure, Ascorbinsäure und gegebenenfalls Thiamin als Feststoffe enthält.

5. Stoffgemisch nach Anspruch 4, wobei das Stoffgemisch in Dosierungseinheiten bereitgestellt wird, die
zwischen 40 g und 250 g, vorzugsweise zwischen 60 g und 200 g Ribose,
zwischen 5 g und 50 g, vorzugsweise zwischen 10 g und 40 g Alanin, zwischen 3 g und 30 g, vorzugsweise zwischen 5 g und 25 g Nikotinsäure,
zwischen 5 g und 75 g, vorzugsweise zwischen 10 g und 50 g Ascorbinsäure, und, gegebenenfalls
zwischen 2 g und 8 g, vorzugsweise zwischen 4 g und 6 g Thiamin enthalten.

6. Stoffgemisch nach einem der Ansprüche 1 bis 3, wobei es sich um ein flüssiges Stoffgemisch handelt, das Ribose, Alanin, Nikotinsäure, Ascorbinsäure und gegebenenfalls Thiamin in wässriger Lösung enthält.

7. Stoffgemisch nach Anspruch 6, wobei die wässrige Lösung
zwischen 40 g/l und 250 g/l, vorzugsweise zwischen 60 g/l und 200 g/l Ribose,
zwischen 5 g/l und 50 g/l, vorzugsweise zwischen 10 g/l und 40 g/l Alanin,
zwischen 3 g/l und 30 g/l, vorzugsweise zwischen 5 g/l und 25 g/l Nikotinsäure, und
zwischen 5 g/l und 75 g/l, vorzugsweise zwischen 10 g/l und 50 g/l Ascorbinsäure, und, gegebenenfalls
zwischen 2 g/l und 8 g/l, vorzugsweise zwischen 4 g/l und 6 g/l Thiamin enthält.

8. Infusionslösung, **dadurch gekennzeichnet, dass** die Infusionslösung die Inhaltsstoffe eines flüssigen Stoffgemisches nach Anspruch 7 in 25-facher oder 50-facher Verdünnung enthält.

9. Infusionslösung nach Anspruch 8, wobei die Infusionslösung zusätzlich zumindest einen homöopathischen Wirkstoff enthält.

10. Verwendung eines Stoffgemisches nach einem der vorangehenden Ansprüche zur Herstellung eines Medikaments oder Nahrungsergänzungsmittels, vorzugsweise einer Infusionslösung, und weiter vorzugsweise einer Infusionslösung nach einem der Ansprüche 8 oder 9.
